(19) Europäisches Patentamt / European Patent Office / Office européen des brevets

(11) **EP 1 920 744 B1**

(12) **EUROPEAN PATENT SPECIFICATION**

(45) Date of publication and mention
of the grant of the patent:
**19.12.2012 Bulletin 2012/51**

(21) Application number: **06796323.1**

(22) Date of filing: **10.08.2006**

(51) Int Cl.:
*A61F 13/49* (2006.01)   *A61F 5/44* (2006.01)
*A61F 13/15* (2006.01)   *A61F 13/53* (2006.01)

(86) International application number:
**PCT/JP2006/315824**

(87) International publication number:
**WO 2007/026526 (08.03.2007 Gazette 2007/10)**

(54) **ABSORBENT SHEET**

ABSORBIERENDE LAGE

FEUILLE ABSORBANTE

(84) Designated Contracting States:
**DE FR GB SE**

(30) Priority: **29.08.2005   JP 2005247672**

(43) Date of publication of application:
**14.05.2008   Bulletin 2008/20**

(73) Proprietor: **Kao Corporation**
**Chuo-ku**
**Tokyo 103-8210 (JP)**

(72) Inventors:
• **YASUDA, Michio**
**Tochigi 3213497 (JP)**

• **KIMURA, Mayumi**
**Tochigi 3213497 (JP)**
• **YAMAMOTO, Yasuhiro**
**Tochigi 3213497 (JP)**

(74) Representative: **Vossius & Partner**
**Siebertstrasse 4**
**81675 München (DE)**

(56) References cited:
**EP-A1- 0 394 812        EP-A2- 0 783 877**
**WO-A1-92/14430        WO-A1-03/030954**
**JP-A- 08 229 070        JP-A- 54 139 981**
**JP-A- 2000 516 864     US-A- 6 086 950**

## Description

Technical Field

The present invention relates to an absorbent sheet suited to absorb various liquids.

Background Art

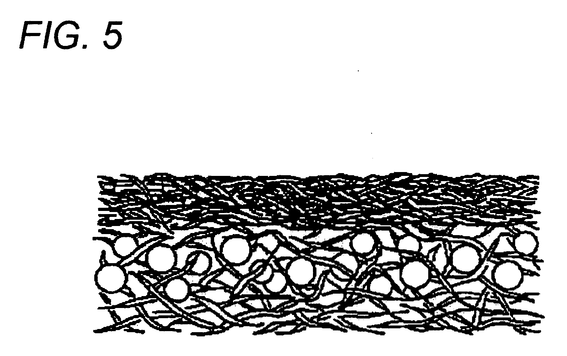

The inventors of the present invention previously proposed in U.S. Patent 6,086,950 an absorbent sheet having superabsorbent polymer particles distributed inside the sheet. The structure of the absorbent sheet proposed is as shown in Fig. 5, in which superabsorbent polymer particles are immobilized by adhesion to hydrophilic fibers constituting the absorbent sheet. The absorbent sheet is advantageous in that the superabsorbent polymer particles, being securely fixed inside the sheet, hardly fall off. Moreover, because the superabsorbent polymer particles have reduced gel-blocking characteristics, the absorbent sheet exhibits a high liquid absorption rate and excellent liquid immobilization properties.

The absorbent sheet of U.S. Patent 6,086,950 is obtained by spreading superabsorbent polymer particles on a wet fiber web as formed by wet papermaking process, overlaying dry paper thereon, and drying the laminate into a unitary sheet. Since the fibers of the wet fiber web have high freedom, the polymer particles spread thereon are three-dimensionally embedded in the interfiber spaces.

If the fibers of the wet fiber web have undue freedom, too many fibers are liable to adhere to the polymer particles being embedded in the interfiber spaces. This favors fixation of the superabsorbent polymer particles but leaves room for further improvement to prevent gel-blocking and to increase the rate of liquid penetration.

WO 92/14430 A1 describes an absorbent product, for use in absorbent articles, comprising an absorbent layer and a wicking layer of densified hydrophilic material. The absorbent layer comprises resilient hydrophilic fibers and superabsorbent material. The absorbent layer includes at least one area comprising a bulky fibrous structure having high void regions of low fiber density randomly distributed in the structure.

EP 0 394 812 A1 provides a unitary absorbent structure with an absorbent pad that includes at least an absorbing layer (B) of absorbent material for absorbing and holding body fluids. A facing layer (A) of body fluid-pervious material is placed the adjacent the absorbing pad. The facing layer (A) and absorbing layer (B) are positioned in face-to-face contact with the contacting faces bonded together over substantially the entire contact area. A fluid-distributing layer (C) may be adhered to one surface of the absorbing layer (B). A backing sheet (D) may be disposed adjacent the absorbent pad, and edge portions of the backing sheet (D) may be wrapped around the edges of the structure and heat sealed to the facing layer (A).

EP 0783 877 A2 describes an absorbent article which comprises at least a liquid retentive absorbent member and a liquid impermeable antileakage layer.

WO 03/030954 A 1 is directed to an absorbent product including an absorbent composite containing superabsorbent material. The superabsorbent material is in the form of superabsorbent particles having a bimodal particle size distribution. Use of superabsorbent material having a bimodal particle size distribution in the absorbent structure results in enhanced capillary driven fluid distribution and enhanced fluid intake of the absorbent composite. The absorbent product may be in the form of a disposable personal care product.

Disclosure of the Invention

The present invention provides an absorbent sheet as defined in the annexed claims including a fibrous material and superabsorbent polymer particles. The superabsorbent polymer particles are held by the fibrous material and distributed in the thickness direction of the absorbent sheet. The superabsorbent polymer particles are not substantially exposed on the surface of the absorbent sheet. The absorbent sheet contains, around at least part of the superabsorbent polymer particles, voids having size sufficient to accommodate a swell (increase in volume) of the polymer particles due to liquid absorption.

Brief Description of the Drawings

Fig. 1 schematically illustrates a cross-sectional structure of an embodiment of the absorbent sheet according to the present invention.
Fig. 2 is a micrograph showing a cut area of the absorbent sheet according to the present invention.
Fig. 3 is a micrograph showing a cut area of a comparative absorbent sheet.
Fig. 4 presents a process chart illustrating a preferred process of producing the absorbent sheet of Fig. 1.

Fig. 5 schematically illustrates a cross-sectional structure of a conventional absorbent sheet.

Best Mode for Carrying out the Invention

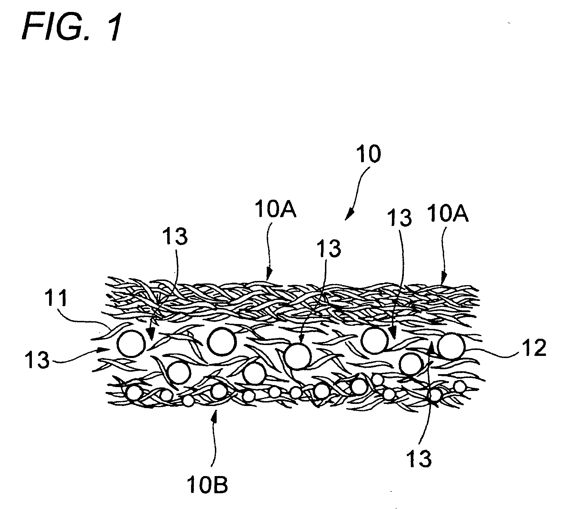

The present invention will be described based on its preferred embodiment by way of the accompanying drawings. Fig. 1 schematically illustrates a cross-sectional structure of an embodiment of the absorbent sheet according to the present invention.

An absorbent sheet 10 is a thin sheet with a first side 10A and a second side 10B. The absorbent sheet 10 is designed to absorb liquid from whichever side, 10A or 10B. That is, the absorbent sheet 10 has an absorbing surface on both the first side 10A and the second side 10B.

The absorbent sheet 10 has a fibrous material 11 and superabsorbent polymer particles (hereinafter also referred to as "polymer particles") 12 as constituents. While the absorbent sheet 10 illustrated in Fig. 1 appears to have a great difference in density of the fibrous material 11 between the first side 10A and the second side 10B, the difference is shown exaggerated for better understanding of the invention. The difference in density of the fibrous material 11 is not so much as depicted in actual absorbent sheets.

As is apparent from Fig. 1, the polymer particles 12 are substantially absent on either side of the absorbent sheet 10. The phrase "substantially absent" as used herein means that the polymer particles 12 are not intentionally disposed on the surface of the absorbent sheet 10. Existence of a trace amount of the polymer particles 12 on the surface of the sheet 10 unavoidably caused in the production of the absorbent sheet 10 is allowable.

The polymer particles 12 are embeddedly supported inside the thickness of the absorbent sheet 10, while being held by the fibrous material 11. The polymer particles 12 are dispersively disposed in the thickness direction of the absorbent sheet 10. In other words, the polymer particles 12 are three-dimensionally distributed inside the absorbent sheet 10. The fibrous material 11 holds the polymer particles 12 by tackiness of the polymer particles 12 developed upon liquid absorption. Being so held, the polymer particles 12 are secured inside the sheet 10 and effectively prevented from falling off. Accordingly, the absorbent sheet 10 of the present embodiment is allowed to contain a large quantity of the polymer particles 12. For example, the absorbent sheet 10 of the present embodiment is capable of containing 15% to 70% by weight, preferably 25% to 65% by weight, of the polymer particles 12 based on its total weight. In addition, the above-described state of the polymer particles being held helps liquid absorbed from the surface of the sheet 10 to reach the polymer particles 12 smoothly.

The absorbent sheet 10 of the present embodiment is characterized by having voids 13 around at least part of the polymer particles 12. The voids 13 have a size (volume) sufficient to accommodate swell volume increase of the polymer particles due to liquid absorption. There is sparse adhesion of fibrous material 11 to the surface of polymer particles 12 having a void 13 therearound. To put it another way, there is a less amount of the fibrous material 11 around the polymer particles 12 having a void 13 therearound to provide a lower apparent density than in other sites in the absorbent sheet 10.

When the polymer particles 12 swell on liquid absorption to increase their volume, the voids 13 formed around the particles 12 accommodate the volume increase so that gel-blocking of the particles 12, i.e., hindrance to swelling is prevented effectively. Furthermore, the voids 13 allow for formation of a coarse network by the fibrous material 11 around the polymer particles 12. The coarse network structure promotes rapid passage of liquid in the thickness direction of the absorbent sheet 10. In particular, the absorbent sheet 10 of the present embodiment hardly suffers from gel blocking and retains a high rate of liquid penetration even after repeated liquid absorption.

Formation of the voids 13 and their size can be confirmed by, for example, microscopic observation on a cut area of the absorbent sheet 10. The size of the voids 13 depends on the process of making the absorbent sheet 10, the size of the polymer particles 12, the kind and fiber length of the fibrous material 11, and the like. In the case where, in an microscopically enlarged cross-section of the absorbent sheet 10, there is a space between polymer particles where no fiber exists, where the fiber density is apparently sparse compared with that in the other sites, or where the fiber density is so sparse as to be virtually non-existent, and the maximum size of which in the cross-sectional thickness direction is at least 25% of the diameter of any adjacent polymer particle, such a space can be defined to be a void 13 in the embodiment.

The absorbent sheet 10 of the present invention does not need to have voids 13 formed around all the polymer particles 12. Desired effects as expected will be exhibited only if 40% to 95%, preferably 60% to 95%, of the number of the polymer particles 12 have the void 13 therearound. Measurement of the voids 13 can be made by observing a cut area of the absorbent sheet 10 under a microscope. An example is described hereunder for illustrative purposes only but not for limitation.

An absorbent sheet to be measured is cut with a sharp razor blade to prepare a 1 cm by 1 cm specimen. The specimen is set on an aluminum stage of an SEM so that the cut area may be observed. Eight sites having a sparse fiber density adjacent to the polymer and appearing to be a void are chosen from an SEM image at a magnification of 100 times and photographed. The size of the sites (voids) with sparse fiber distribution is measured with the naked eye using a ruler. SEM observation may be replaced with observation under an optical microscope.

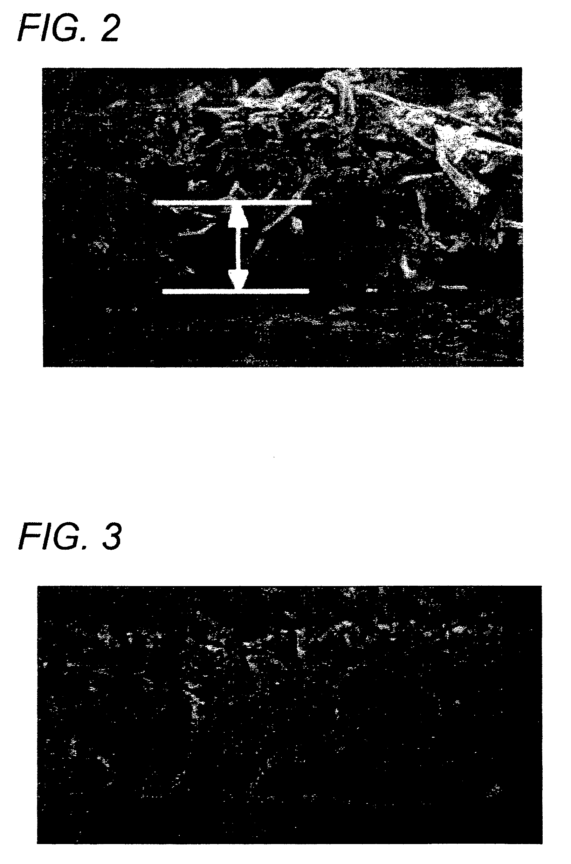

Fig. 2 is a micrograph of an exemplary absorbent sheet of the present invention as observed in the manner described. The range indicated by the two-headed arrow in Fig. 2 is a void. Fig. 3 shows a comparative absorbent sheet (the sheet illustrated in Fig. 5), in which no void is observed.

The polymer particles 12 have a size distribution so as to be composed of particles of various sizes. Large particles will facilitate forming the voids 13 therearound, and small particles will be embedded into the lower layer to bring about improved absorbency. For easy formation of the voids 13 around the particles 12, 40% by weight or more, preferably 60% by weight or more, of the particles 12 have a particle size of 250 $\mu$m or greater. The term "particle size" of the polymer particles 12 means a diameter when a particle is spherical, or the largest opening size of a sieve through which particles can pass in classification when a particle is aspherical, e.g., irregular in shape. Size distribution measurement (hereinafter referred to as classification) of the polymer particles 12 is carried out as follows. In the first classification step, the polymer particles 12 are size classified using test sieves with 106 $\mu$m, 150 $\mu$m, and 500 $\mu$m openings, respectively, available from Tokyo Screen Co., Ltd. into four size fractions: -106 $\mu$m, +106/-150 $\mu$m, +150/-500 $\mu$m, and +500 $\mu$m. In the second step, the +150/-500 $\mu$m fraction is further classified using test sieves with 250 $\mu$m and 355 $\mu$m openings, respectively, into three fractions in order to identify the particle sizes participating in the void formation. In the first classification step for measuring rough distribution, the width of the size distribution of the polymer particles 12 may be about 50 to 500 $\mu$m. In the second classification step for identifying the particle sizes participating in the void formation, the width is preferably 30 to 150 $\mu$m, more preferably 50 to 100 $\mu$m.

While the particle size distribution of the polymer particles 12 is as stated previously, the average particle size of the particles 12 is preferably 150 to 500 $\mu$m, more preferably 250 to 350 $\mu$m, from the standpoint of ease of the void formation around the particles 12. The term "average particle size" as used herein denotes a value obtained by multiplying an average of two opening sizes by the weight fraction of the polymer particles falling in the range between the two sizes as derived from the results of the aforementioned sieve method. For example, the average of opening sizes 150 $\mu$m and 250 $\mu$m is 200 $\mu$m, which is multiplied by the weight fraction of the polymer derived from the results of classification. The average of -106 $\mu$m opening sizes is assumed to be 53 $\mu$m. The +500 $\mu$m fraction is further classified to reduce the weight fraction to less than 5%. Polymer particles whose weight fraction becomes less than 5% are excluded from calculation.

The polymer particles 12 have a size distribution, Such that the particle size of the polymers 12 gradually decreases from one side of the absorbent sheet 10 to the other, e.g., from the first side 10A to the second side 10B or *vise versa.* The greater the polymer particles 12, the more the voids 13 are formed. Conversely, the smaller the polymer particles 12, the more the amount of the fibrous material 11 adhered thereto. In Fig. 1, the size of the polymer particles 12 gradually decreases from the first side 10A toward the second side 10B. With the particles 12 being so distributed, gel blocking is prevented more effectively, and the rate of liquid penetration is further increased.

The particulate polymer 12 can have various shapes including spheres, blocks, spindles, and irregular shapes depending on the production process. For ease of forming the voids 13 around the polymer particles 12, it is preferred to use polymer particles 12 having shape anisotropy, particularly irregular-shaped particles 12, for the following reason. To form spaces, it is important that the polymer particles 12 not be buried in the fibrous material 11. The fibrous material 11 is a porous material with discontinuous pores. With the size of the pores being equal, irregular-shaped polymer particles 12 are less likely to be buried in the fibrous material 11 than spherical ones. Furthermore, irregular-shaped polymer particles 12 are more apt to secure spaces between them than spherical ones. It is therefore preferred to use irregular-shaped polymer particles 12.

Any conventional superabsorbent polymer can be used as the particulate polymer 12. Examples of suitable polymers include starch, crosslinked carboxymethyl cellulose, polyacrylic acids and the salts thereof, such as a homo- or copolymer of acrylic acid or its alkali metal salt, and polyacrylic acid salt graft polymers. The polyacrylic acid salts preferably include a sodium salt. Also preferred are acrylic acid copolymers containing a comonomer unit derived from maleic acid, itaconic acid, acrylamide, 2-acrylamido-2-methylpropanesulfonic acid, 2-(meth)acryloylethane-sulfonic acid, 2-hydroxyethyl (meth)acrylate, styrenesulfonic acid, etc. in such a proportion that does not impair the performance of superabsorbent acrylic acid polymers.

The fibrous material 11 holding the polymer particles 12 is preferably hydrophilic to enhance liquid penetration and absorption characteristics. Examples of such fibers include, but are not limited to, natural cellulose fibers such as wood pulp (e.g., softwood or hardwood kraft pulp), cotton pulp, and straw pulp, regenerated cellulose fibers such as rayon and cuprammonium, hydrophilic synthetic fibers such as polyvinyl alcohol fiber and polyacrylonitrile fibers, and synthetic fibers (e.g., polyethylene fiber, polypropylene fiber, and polyester fiber) having been hydrophilized with a surface active agent. These hydrophilic fibers can be used either individually or as a combination of two or more thereof.

Cellulosic fibers are preferred of the hydrophilic fibers. Bulky cellulosic fibers, such as natural cellulose fibers and regenerated cellulose fibers, are particularly preferred. Wood pulp, especially softwood kraft pulp, is preferred from economical viewpoint. Such bulky cellulosic fibers exhibit improved capabilities of dispersing and immobilizing the polymer particles 12. Moreover, the bulky cellulosic fibers help the polymer particles 12 to be embedded, dispersed, and immobilized in three dimensions. Furthermore, the bulky cellulosic fibers are effective in suppressing gel blocking. For all these considerations, the bulky cellulosic fibers are preferably used in a proportion of 40% to 95% by weight, more preferably 50% to 80% by weight, based on the weight of the absorbent sheet 10. The terminology "bulky fibers" as used herein means fibers having a three-dimensional form, such as a twist, a crimp, a bend and/or a branch, or having a very large thickness (e.g., a fiber coarseness of 0.3 mg/m or more).

One preferred example of the bulky cellulosic fibers is those having a fiber coarseness of 0.3 mg/m or more. Cellulosic fibers having a fiber coarseness of 0.3 mg/m or more can be accumulated while retaining their bulkiness. Therefore, they can provide a bulky network structure in which the polymer particles 12 are held. In such a bulky network structure, a liquid can migrate at a higher speed, encountering less resistance. The fiber coarseness of the bulky cellulosic fiber is preferably 0.3 to 2 mg/m, more preferably 0.32 to 1 mg/m.

"Fiber coarseness" is a measure used to represent the thickness of fiber with an uneven thickness such as wood pulp. Measurement of fiber coarseness can be taken with, for example, a fiber length analyzer FS-200 available from Kajanni Electronics, Ltd.

Examples of the cellulosic fibers having a fiber coarseness of 0.3 mg/m or more is softwood kraft pulp (ALBACEL from Federal Paper Board Co. and INDORAYON from PT Inti Indoraryon Utama).

Another preferred example of the bulky cellulosic fibers is those having a cross-section with a circularity of 0.5 to 1, preferably 0.55 to 1. Cellulosic fibers with a circularity of 0.5 to 1 cause little resistance to liquid migration to secure a high rate of liquid penetration. Circularity of fiber is measured as follows. A fiber is sliced vertically in a direction perpendicular to its length, and the cut area is electron-microphotographed. The micrograph is analyzed on an image analyzer New Qube ver. 4.20 from Nexus to measure the circumference and the cross-sectional area of the fiber. The circularity of the fiber is calculated from equation:

$$\text{Circularity} = 4\pi \, (\text{cross-sectional area})/(\text{circumference})^2$$

Measurements are made on randomly chosen 100 cut areas to obtain an average circularity.

As stated, wood pulp is preferred cellulosic fiber. Wood pulp, however, generally has a flattened cross-section as a result of delignification, having a circularity of less than 0.5. Such flat wood pulp fibers can be converted to fibers with a circularity of 0.5 or more by mercerization (swell).

That is, mercerized pulp fibers with a circularity of 0.5 to 1 which is obtained by mercerization of wood pulp are also preferred bulky cellulosic fibers. Commercially available mercerized pulps that can be used in the invention include FILTRANIER and POROSANIER, both from ITT Rayonier, Inc.

Cellulosic fibers having both a coarseness of 0.3 mg/m or more and a cross-sectional circularity of 0.5 to 1 are more preferred for their capability of forming a bulky network structure more easily and achieving a higher rate of liquid penetration.

Still another preferred example of the bulky cellulosic fibers is crosslinked cellulose fibers having an intramolecular and/or intermolecular crosslinked structure. Such crosslinked cellulosic fibers are preferred as being capable of maintaining the bulkiness even in a wet state. Commercially available crosslinked cellulose fibers include High Bulk Additive from Weyerhaeuser Paper Co.

In addition to the bulky cellulosic fibers described above, also preferred are crosslinked cellulosic fibers obtained by intramolecularly and/or intermolecularly crosslinking cellulosic fibers (e.g., pulp) having a fiber coarseness of 0.3 mg/m or more or a cross-sectional circularity of 0.5 to 1. Also preferred are crosslinked pulp fibers obtained by intramolecularly and/or intermolecularly crosslinking mercerized pulp fibers having a cross-sectional circularity of 0.5 to 1. More preferred are those obtained by crosslinking pulp fibers having both a fiber coarseness of 0.3 mg/m or more and a cross-sectional circularity of 0.5 to 1. Even more preferred are those obtained by mercerizing pulp fibers having a coarseness of 0.3 mg/m or more to increase the circularity to 0.5 to 1, followed by crosslinking.

It is also preferred to use heat fusible fibers that melt on heating and bond to each other as the fibrous material 11 in combination with the above-described bulky cellulosic fibers. Using heat fusible fibers is effective in imparting sufficient wet strength to the absorbent sheet 10. Examples of useful heat fusible fibers include polyolefin fibers such as polyethylene, polypropylene, and polyvinyl alcohol, polyester fibers, polyethylene-polypropylene conjugate fibers, polyethylene-polyester conjugate fibers, low-melting polyester-polyester conjugate fibers, polyvinyl alcohol-polypropylene conjugate fibers having a hydrophilic surface, and polyvinyl alcohol-polyester conjugate fibers having a hydrophilic surface. The conjugate fibers may have a sheath/core configuration or a side-by-side configuration. These heat fusible fibers can be used either individually or as a mixture of two or more thereof. The heat fusible fibers preferably have a fiber length of 2 to 60 mm and a fineness of 0.1 to 3 denier, more preferably 0.5 to 3 denier. The heat fusible fibers are preferably used in a proportion of 1% to 50% by weight, more preferably 3% to 30% by weight, based on the total weight of the absorbent sheet 10.

The fiber length of the fibrous material 11 is not critical in the present invention. When the absorbent sheet 10 is fabricated by, for example, a wet papermaking technique as described *infra,* a preferred fiber length of the fibrous material 11 is 1 to 20 mm.

The absorbent sheet 10 can further contain a polyamine epichlorohydrin resin, dialdehyde starch, carboxymethyl cellulose, etc. as a wet-strength additive.

The absorbent sheet 10 of the present embodiment is characterized by containing the polymer particles 12 in a high proportion and yet having a small thickness. Specifically, the absorbent sheet 10 is as thin as 0.4 to 2 mm, preferably 0.5 to 1.5 mm, with its weight falling within a range of from 50 to 150 $g/m^2$, more preferably 60 to 120 $g/m^2$.

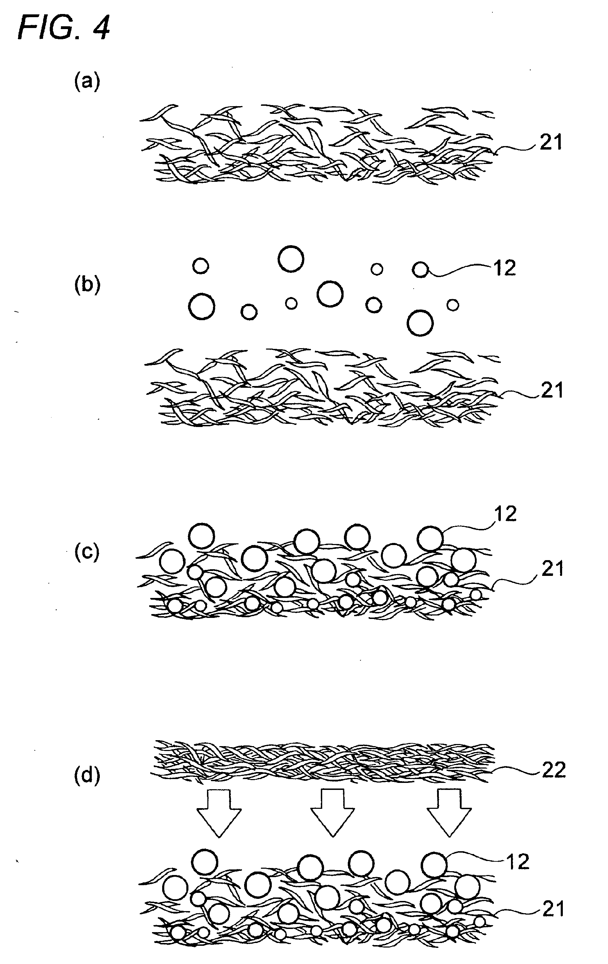

A preferred process of producing the absorbent sheet 10 according to the present embodiment will now be described by referring to Fig. 4. As illustrated in Fig. 4(a), a first paper layer 21 is formed by a wet papermaking technique. The first paper layer 21 preferably contains the above-described bulky cellulosic fibers and/or heat fusible fibers. The first paper layer 21 used in the present embodiment must have the constituent fibers tightly constrained by each other, i.e., bound to each other. Because of the tight binding of the fibers, the first paper layer 21 does not allow polymer particles 12 to easily sink inside thereof, which facilitates void formation. Such a paper layer is exemplified by a sheet in which the fibers are bound together with a binder, etc. and thereby have limited freedom.

The bulk of the paper layer 21 may be used as a measure of the binding force of fibers. To have a small bulk means that the fibers are closely packed, indicating strong constraint of the fibers. To have a high bulk means that the fibers are loosely packed, indicating weak binding force of the fibers. The bulk of the first paper layer 21 is obtained by dividing the grammage of the paper layer 21 by the thickness. The first paper layer 21 having a bulk of 0.04 to 1.5 $g/cm^3$, preferably 0.06 to 1.5 $g/cm^2$, can be said to have the fibers bound together sufficiently tightly.

A predetermined amount of water is sprayed on the first paper layer 21 to wet the paper. On being sprayed with water, the fibers making up the first paper layer 21 loosen their hydrogen bonds and are disentangled to reduce the binding force. As a result, the interfiber distance increases to such a degree as to be capable of receiving the polymer particles 12. To achieve moderate reduction of the binding force, the amount of water to be supplied is preferably 30% to 200% by weight, more preferably 60% to 100% by weight, based on the weight of the first paper layer 21 in a dry state.

Polymer particles 12 are then spread on the thus wetted first paper layer 21 as illustrated in Fig. 4(b). The polymer particles 12 may be uniformly distributed over the entire surface of the first paper layer 21 or fed in a pattern of stripes continuously or discontinuously extending in parallel in one direction. The spread polymer particles 12 embed themselves into the widened interfiber spaces. In the case where the particle size distribution of the polymer particles 12 has a certain breadth, as shown in Fig. 4(c), the first paper layer 21 with the widened interfiber distance acts like a sieve to let smaller polymer particles 12 embed themselves deeper and, conversely, to make greater polymer particles 12 remain on or nearer the surface of the first paper layer 21. That is, the particle size of the polymer particles 12 gradually decreases in a vertically downward direction in Fig. 4(c). The gradual decrease in particle size is especially conspicuous when the polymer particles 12 have shape anisotropy, particularly when polymer particles 12 having an irregular shape are used. It is also conspicuous as a result of using polymer particles 12 having a size distribution and containing particles of 250 $\mu$m or greater in a weight proportion of 40% or more.

The embedded polymer particles 12 absorb the water content of the first paper layer 21 to swell and develop tackiness, by which they adhere to the constituent fibers of the first paper layer 21.

According to the process of producing an absorbent sheet described in U.S. Patent 6,086,950 cited *supra,* polymer particles are spread on a wet fiber web as formed on the forming wire of a paper machine. Comparing the binding force of fibers in the wet fiber web and that in the wetted first fiber layer 21, the first fiber layer 21 prepared by once drying and then wetting can be seen as having a stronger binding force of fibers. Although the polymer particles 12 spread on the wet first paper layer 21 develops tackiness and adhere to the fibers, the binding force between fibers in the first paper layer 21 is not so weak as to permit the fibers to be drawn from the first paper layer 21 and to adhere to the polymer particles 12 in a considerable amount. In other words, the fibers show a reduced tendency to adhere to the surface of the polymer particles 12 spread on the wet first paper layer 21. The reduction in tendency to adhesion is more notable with larger polymer particles 12 positioned nearer to the surface of the first paper layer 21. It follows that the fibers are sparse around large polymer particles 12 existing on and near the surface of the first paper layer 21 as illustrated in Fig. 4(c).

After completion of the spread of the polymer particles 12, a second paper layer 22 is superposed on the polymer-spread side of the first paper layer 21 as illustrated in Fig. 4(d). Similarly to the first paper layer 21, the second paper layer 22 preferably contains the aforementioned bulky cellulosic fibers and/or heat fusible fibers. The fiber composition may be the same or different between the first paper layer 21 and the second paper layer 22. A highly bulky fiber sheet is preferred as the second paper layer 22 for ease of holding relatively small polymer particles.

As previously stated, there are large polymer particles 12 on the polymer-spread side of the first paper layer 21, and the fibers are sparse around the large polymer particles 12. When the second paper layer 22 is overlaid on the polymer spread side of the first paper layer 21 in that state, the large particles 12 serve as pillars or spacers to secure a certain amount of spaces between the first paper layer 21 and the second paper layer 22. In this state, the first paper layer 21 and the second paper layer 22 are independent of each other, just making a two-ply structure. The whole of the two-ply structure is then subjected to heat drying simultaneously with, or followed by, light pressing, whereby the first paper layer 21 and the second paper layer 22 are joined together via the polymer into a unitary sheet, i.e., an apparently single-ply sheet. Meanwhile, entanglement and hydrogen bond formation occur in parts between the fibers of the first paper layer 21 and those of the second paper layer 22 to ensure the integrity. The absorbent sheet 10 as desired is thus obtained. While the two layers 21 and 22 are being joined together, the large polymer particles 12 present between the two layers 21 and 22 continue serving as spacers to retain spaces therearound, which spaces become the voids 13 shown in Fig. 1.

The temperature of drying the superposed layers 21 and 22 is preferably 100° to 180°C, more preferably 105° to 150°C. To carry out the pressing, the least pressure required to unify the two layers will be enough. The lower the pressure, the more the spaces secured.

To help better understanding of the liquid absorption mechanism in the absorbent sheet 10, the mechanism will hereinafter be explained using a model. Polymer particles 12 having various sizes are separated into relatively large particles remaining on the surface of the first paper layer 21 and relatively small particles entering inside the first paper layer 21. The separation is governed by the interfiber distance in the first paper layer 21. Therefore, it is possible to decide the particle size of the polymer particles that are to remain on the surface of the first paper layer 21 by selecting the first paper layer 21. The amount of the polymer particles to be left on the first paper layer 21 is decided with consideration given to the prevention of gel blocking that is a cause of absorbency reduction. The amount of the polymer to be spread is then decided. A suitable distance between the polymer particles varies depending on the liquid to be absorbed. For example, in the case of a bodily fluid that can be absorbed in relatively large quantities, such as urine and perspiration, the polymer particles are spread considering that they may swell about 150 to 300 times their initial volume. In the case of such a body fluid as menstrual blood or blood, the polymer particles are spread considering that they may swell about 5 to 50 times their initial volume. In the latter case, assuming that spherical polymer particles with a uniform particle diameter (r) arranged on the first paper layer 21 in a regular lattice pattern swell 30 times the initial volume on absorbing artificial blood, it is only necessary that the spherical polymer particles arranged in a lattice pattern be at a center-to-center distance of the cube root of 30 multiplied by r. Actually it is very difficult to arrange the polymer particles in such an ideal pattern. According to the process of producing an absorbent sheet disclosed in U.S. Patent 6,086,950, since polymer particles are spread on a wet fiber web as formed by wet papermaking, the polymer particles swell while moving so as to avoid coming close to each other where possible, whereby gel blocking does not occur, and after forming the absorbent sheet, the sheet is dried to form spaces around the polymer particles. It is preferred to form a pattern of minute depressions on the lower sheet so that the polymer particles may not be dispersively fixed.

The following is another preferred process of producing the absorbent sheet 10. The process starts with preparation of a first paper layer. The first paper layer can be prepared in the same manner as described above. Polymer particles are then spread on the paper layer. A second paper layer is overlaid thereon. The second paper layer can be prepared in the same manner as described above. Before being overlaid, water is supplied to the second paper layer to make it wet. The wet second paper layer is placed on the polymer spread side of the first paper layer, and the stack is subjected to heat drying simultaneously with or followed by pressing to obtain the absorbent sheet 10.

The following process is also preferably adopted to produce the absorbent sheet 10. The process starts with preparation of a first paper layer. The first paper layer can be prepared in the same manner as described above. One side of the first paper layer is napped (fuzzed) by, for example, using a napping roller having barbed or hooked needles or by inserting needles into and withdrawing from the paper layer like needle-punching. Polymer particles are spread on the napped surface, and a second paper layer is overlaid thereon. The second paper layer can be prepared in the same manner as described *supra*. Thereafter, a predetermined amount of water is sprayed. Alternatively, water is sprayed on the polymer spread side of the first paper layer, and the second paper layer is then overlaid thereon. The stack of the layers 21 and 22 are subjected to heat drying simultaneously with or followed by pressing to give the absorbent sheet 10.

The following is still another preferred process of producing the absorbent sheet 10. A web as a first paper layer 21 is formed out of the above-mentioned bulky cellulosic fibers and/or heat fusible fibers by carding or airlaying. Polymer particles 12 are spread on the web of the first paper layer 21, a second paper layer 22 overlaid thereon, and the stack unified. In the step of spreading the polymer particles 12 on the web of the first paper layer 21, the polymer particles 12 spread on the web is distributed in the thickness direction of the first paper layer 21 according to their particle size by intentionally vibrating or sucking the web while being transferred. When the polymer particle 12 are spread while sucking the web of the first paper layer 21, even if some particles pass through the web, such particles are removed from the other side (outer side) of the first paper layer 21 by suction, leaving no polymer particles 12 on the outer side of the first paper layer 21. In order to control the disperse state of the polymer particles 12, it is preferred that the interfiber distance be adjusted before spreading the polymer particles 12 by, for example, introducing the first paper layer 21 into the nip of a pair of rolls.

Unifying the two webs is preferably performed by the following methods. In the first method, water is sprayed on the web on which the polymer particles 12 have been spread, and the second paper layer 22 is overlaid, or water is sprayed on the second paper layer 22 having been overlaid on the polymer spread side of the web, whereby the polymer particles 12 absorb water to develop tackiness. The laminate is then heat dried into a unitary sheet. In the second method, the web having the polymer particles 12 spread thereon or the second fiber layer 22 is spray coated with an adhesive or coated with a pressure-sensitive adhesive such as a hot melt adhesive in a dot, a linear, wavy or helical pattern, followed by press-bonding under heat to provide a unitary sheet. The method of unifying is not limited to the above-described examples, and other unifying means can be employed. For example, a binder fiber or component may be incorporated into the first paper layer 21 and/or the second paper layer 22, and the laminate is heated to activate the binder fiber or component to adhere the two paper layers.

The absorbent sheet 10 thus produced has a small thickness and yet high absorption capacity and is therefore suited for use as an absorbent member in various hygienic personal care products including sanitary napkins, disposable diapers, hygienic pads, medical pads, and nursing pads; drip sheets, kitchen paper towel, household cleaning sheets, pet sheets or pads, and the like. The absorbent sheet 10 is designed to absorb liquid from whichever side. Since the polymer particles 12 gradually decrease in particle size from one side to the other of the absorbent sheet 10, using the side with larger particle sizes as an absorbing surface reduces gel blocking of the polymer particles 12 and enjoys an increased rate of liquid penetration.

While the invention has been described in detail with reference to its preferred embodiment, it should be understood that the invention is not limited thereto, and various changes and modifications can be made therein as will be apparent to those skilled in the art.

Examples

The present invention will now be illustrated in greater detail by way of Examples. It should be understood that the invention is not limited thereto. Unless otherwise noted, all the parts and percents are given by weight.

Example 1

An absorbent sheet shown in Fig. 1 was made in accordance with the process illustrated in Fig. 4. First of all, crosslinked pulp having a fiber coarseness of 0.32 mg/m and a cross-sectional circularity of 0.30 (High Bulk Additive HBA-S, available from Weyerhaeuser Paper Co.) was dispersed in water. A strength additive (polyamide epichlorohydrin resin, Kaimen WS-570 produced by Nippon PMC Co.) was dispersed therein in an amount of 1 part in terms of resin component per 100 parts of the dry weight of the pulp. After the concentration was adjusted, the resulting slurry was formed into a first fiber layer having a dry grammage of 50 g/m$^2$.

The first paper layer was wetted by spraying 100% of water based on the dry weight of the paper layer. Superabsorbent polymer particles (Aqualic W4 from Nippon Shokubai Co., Ltd.) were spread substantially uniformly on the wet first paper layer at an amount of 30 g/m$^2$. The polymer particles had a size distribution in a range of 106-500 μm, in which the weight proportion of the particles of 250 μm or greater was 57%. The average particle size of the polymer particles was 260 μm. The polymer particles had an irregular shape.

A second paper layer having the same grammage and composition as the first paper layer was superposed on the polymer spread side of the first paper layer. The stack of the paper layers was dried by heating at 130°C while being pressed under a relatively low pressure into an integral unitary absorbent sheet having a one-ply structure. The thickness of the sheet was 0.65 mm. Electron microscopic observation on a cut area of the sheet lent confirmation to the structure illustrated in Fig. 1.

EXAMPLE 2

An absorbent sheet was obtained in the same manner as in Example 1, except for using polymer particles having a size distribution in a range of 106-500 μm, in which the weight proportion of the particles of 250 μm or greater was 84%. The average particle size of the polymer particles was 320 μm. The polymer particles had an irregular shape. The thickness of the resulting sheet was 0.72 mm. Microscopic observation on a cut area of the sheet provided confirmation that the sheet had the structure shown in Fig. 1.

COMPARATIVE EXAMPLE 1

An absorbent sheet was obtained in the same manner as in Example 1 with the following exceptions. The polymer particles were spread on a fiber web as formed on the forming wire of a paper machine. The fiber web contained 100 parts of water based on its dry weight. The same second paper layer as used in Example 1 was overlaid on the polymer spread side of the fiber web. The resulting absorbent sheet had a thickness of 0.53 mm. As a result of electron microscopic observation on a cut area of the sheet, many fibers were found adhered to the surface of the polymer particles, and no voids were observed.

Evaluation

The sheets obtained in Examples and Comparative Example were evaluated for rate of absorption as follows. The results obtained are shown in Table 1 below. A 15 cm square test piece was cut out of the sheet. A 10 cm square transparent acrylic plate having a through-hole of 1 cm in diameter in its center was placed on the test piece, and weights were put thereon around the hole to apply a load of 5 g/cm$^2$ to the absorbent sheet 10. Three milliliters of physiological saline was poured through the hole, and the time required for the sheet to absorb physiological saline was measured. One minute later, the same absorption test was repeated to measure the time for re-absorption.
TABLE 1

|  | Example 1 | Example 2 | Comp. Example 1 |
|---|---|---|---|
| 1 st Absorbing Time (sec) | 8 | 7 | 12 |
| 2nd Absorbing Time (sec) | 20 | 14 | 28 |

As is apparent from the results in Table 1, it is seen that the absorbent sheets of Examples do not reduce in rate of absorption in repeated absorption. This means that gel blocking of the polymer particles does not occur in the sheets. In contrast, the comparative absorbent sheet reduces in rate of absorption when repeatedly used, which indicates occurrence of gel blocking of the polymer particles.

Industrial Applicability

As described in detail, the absorbent sheet according to the present invention has voids around superabsorbent polymer particles. The voids accommodate a swell of the polymer particles resulting from liquid absorption and thereby effectively prevent gel blocking. The voids accelerate liquid penetration. A high rate of liquid penetration is sustained even in repeated absorption.

**Claims**

1. An absorbent sheet comprising a fibrous material and superabsorbent polymer particles,
the superabsorbent polymer particles being held by the fibrous material and distributed in the thickness direction of the absorbent sheet, wherein the particle size of the superabsorbent polymers distributed in the thickness direction of the absorbent sheet gradually decreases from one side to the other side of the absorbent sheet, and the greater the polymer particles, the more are the voids formed therearound, and the superabsorbent polymer particles are in a state adhered to the fibrous material by their own tackiness developed on water absorption
the superabsorbent polymer particles being not substantially exposed on the surface of the absorbent sheet, and the absorbent sheet containing, around at least part of the superabsorbent polymer particles, voids having size sufficient to accommodate a swell of the polymer particles due to liquid absorption,
wherein the superabsorbent polymer particles have a particle size distribution in which 40% by weight or more of the particles have a particle size of 250 μm or more.

2. The absorbent sheet according to claim 1, wherein the average particle size of the superabsorbent polymer particles is 150 to 500 μm.

3. The absorbent sheet according to any one of claims 1 or 2, wherein the superabsorbent polymer particles have an irregular shape.

4. The absorbent sheet according to any one of claims 1 to 3, containing 40% to 95% by weight of bulky cellulosic fibers.

5. The absorbent sheet according to any one of claims 1 to 4, having a thickness of 0.4 to 2 mm.

**Patentansprüche**

1. Eine absorbierende Lage, umfassend ein Fasermaterial und Superabsorber-Polymerteilchen,
   wobei die Superabsorber-Polymerteilchen durch das Fasermaterial gehalten und in der Dickenrichtung der absorbierenden Lage verteilt werden, wobei die Teilchengröße der Superabsorber-Polymerteilchen, welche in der Dickenrichtung der absorbierenden Lage verteilt sind, graduell von einer Seite zu der anderen Seite der absorbierenden Lage abnimmt, und wobei, je größer die Polymerteilchen sind, sich desto mehr Hohlräume darum herum bilden, und wobei die Superabsorber-Polymerteilchen sich in einem Zustand befinden, in dem sie durch ihre eigene Klebkraft, welche sich durch Wasserabsorption entwickelt, an dem Fasermaterial anhaften,
   wobei die Superabsorber-Polymerpartikel im Wesentlichen nicht auf der Oberfläche der absorbierenden Lage exponiert sind, und
   wobei die absorbierende Lage um wenigstens einen Teil der Superabsorber-Polymerteilchen herum Hohlräume enthält, welche eine ausreichende Größe haben, um Platz für ein Aufquellen der Polymerteilchen aufgrund von Flüssigkeitsabsorption zu bieten,
   wobei die Superabsorber-Polymerteilchen eine Teilchengrößenverteilung aufweisen, in welcher 40 Gewichts-% oder mehr der Teilchen eine Teilchengröße von 250 $\mu$m oder mehr haben.

2. Die absorbierende Lage nach Anspruch 1, wobei die mittlere Teilchengröße der Superabsorber-Polymerteilchen 150 bis 500 $\mu$m beträgt.

3. Die absorbierende Lage nach einem der Ansprüche 1 oder 2, wobei die Superabsorber-Polymerteilchen eine unregelmäßige Form haben.

4. Die absorbierende Lage nach einem der Ansprüche 1 bis 3, enthaltend 40 Gewichts-% bis 95 Gewichts-% voluminöser Cellulosefasern.

5. Die absorbierende Lage nach einem der Ansprüche 1 bis 4, die eine Dicke von 0,4 bis 2 mm aufweist.

**Revendications**

1. Feuille absorbante comprenant un matériau fibreux et des particules de polymères super-absorbants,
   les particules de polymères super-absorbants étant retenues par le matériau fibreux et réparties dans le sens de l'épaisseur de la feuille absorbante, la taille de particule des polymères super-absorbants répartis dans le sens dé l'épaisseur de la feuille absorbante diminuant progressivement d'un côté à l'autre de la feuille absorbante, et plus les particules de polymères sont grandes, plus il y a de vides formés autour d'elles, et les particules de polymères super-absorbants sont dans un état adhérant au matériau fibreux par leur propre pouvoir collant qui s'ést développée par absorption d'eau,
   les particules de polymères super-absorbants n'étant pratiquement pas exposées sur la surface de la feuille absorbante, et
   la feuille absorbante contenant, autour d'au moins une partie des particules de polymères super-absorbants, des vides ayant une taille suffisante pour s'adapter au gonflement des particules de polymère dû à l'absorption de liquide, dans laquelle les particules de polymères super-absorbants ont une répartition de taille de particule dans laquelle 40 % en poids ou plus des particules ont une taille de particule de 250 $\mu$m ou plus.

2. Feuille absorbante selon la revendication. 1, dans laquelle la taille de particule moyenne des particules de polymères super-absorbants est de 150 à 500 $\mu$m.

3. Feuille absorbante selon l'une quelconque des revendications 1 ou 2, dans laquelle les particules de polymères super-absorbants ont une forme irrégulière.

4. Feuille absorbante selon l'une quelconque des revendications 1 à 3, contenant de 40 % à 95 % en poids de fibres de cellulose volumineuses.

5. Feuille absorbante selon l'une quelconque des revendications 1 à 4, ayant une épaisseur de 0,4 à 2 mm.

## FIG. 1

FIG. 2

FIG. 3

# FIG. 4

(a)

21

(b)

12

21

(c)

12

21

(d)

22

12

21

FIG. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6086950 A
- WO 9214430 A1
- EP 0394812 A1
- EP 0783877 A2
- WO 03030954 A1